# FASCICULE DE BREVET EUROPEEN

(11) **EP 3 581 157 B1**
(45) Date de publication et mention de la délivrance du brevet: **20.01.2021**
(21) Numéro de dépôt: 19172439.2
(22) Date de dépôt: 03.05.2019
(51) Int. Cl.: A61F 13/14, A61F 5/02

(54) **CEINTURE DE SOUTIEN LOMBAIRE**
LENDENSTÜTZGÜRTEL
LUMBAR SUPPORT BELT

(30) Priorité: 11.06.2018 FR 1855053
(43) Date de publication de la demande: 18.12.2019
(73) Titulaire: Gibaud, 42000 Saint Etienne (FR)
(72) Inventeur: DEVILLERS, Laurent, 69126 BRINDAS (FR); DUPORT, Guillaume, 42000 SAINT ETIENNE (FR)
(74) Mandataire: Delorme, Nicolas

(56) Documents cités:
- EP-A1- 3 222 255
- WO-A1-2010/001030
- WO-A1-2013/132180
- FR-A1- 2 944 204

## Description

La présente invention concerne une ceinture de soutien lombaire.

Une telle ceinture est destinée aux personnes souffrant de lombalgie, notamment. Lorsqu'elle est correctement positionnée, la ceinture permet d'une part de diminuer la douleur ressentie par l'utilisateur et d'autre part de maintenir l'utilisateur dans une bonne position, en particulier grâce à la contention de sa région lombaire et de sa région abdominale.

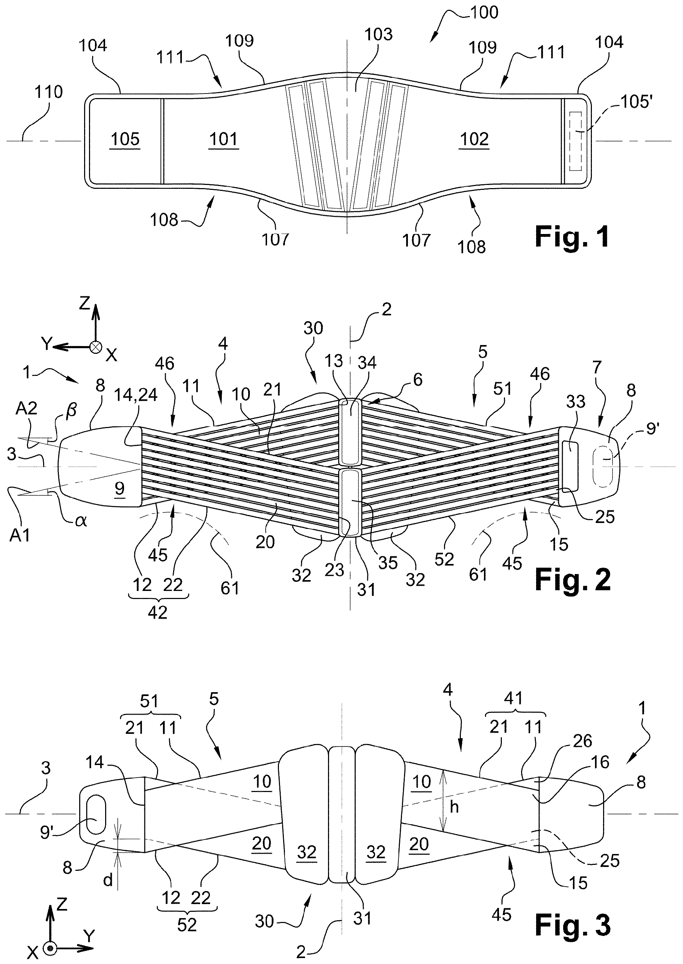

La figure 1 illustre une ceinture 100 de soutien lombaire de l'art antérieur.

La ceinture 100 comporte deux branches 101, 102 destinées à être positionnées de part et d'autre de la partie inférieure du tronc d'un porteur. Les branches 101, 102 sont liées entre elles à leur partie extrême médiale 103, tandis que leurs parties extrêmes latérales 104 sont pourvues de moyens d'accrochage complémentaires 105, 105' pour pouvoir être assemblées lorsqu'elles sont placées l'une sur l'autre en regard de la zone abdominale du porteur Un tel type de ceinture est par exemple décrit dans EP 3 222 255 A1.

De façon conventionnelle, la ceinture 100 présente une partie médiane plus haute que le reste de la ceinture, de façon à former un appui satisfaisant contre le dos du porteur. En outre, le bord inférieur 107 de chaque branche 101, 102 présente une échancrure 108 qui, lorsque la ceinture 100 est portée, se trouve située au-dessus d'une cuisse du porteur. Une telle échancrure 108 permet le passage des cuisses en position assise du porteur, évitant ainsi que la ceinture ne se plie sur la cuisse et/ou qu'elle ne remonte le long du tronc du porteur. La ceinture 100 peut présenter un bord supérieur 109 symétrique du bord inférieur 108 par rapport à un axe longitudinal 110 de la ceinture 100, le bord supérieur 109 présentant ainsi également une échancrure 111 sur chaque branche 101, 102.

Cette forme précitée de la ceinture 100 peut être obtenue à partir d'une unique pièce de textile, comme illustré sur la figure 1. Un tel mode de réalisation requiert toutefois des opérations de confection importantes et donc onéreuses, en particulier des étapes de découpe à la forme appropriée, de couture et de bordage Les documents FR 2 944 204, WO 2010/001030 A1 et WO 2013/132180 divulguent un dispositif de soutien lombaire comprenant une ceinture élastique, ladite ceinture comporte des bandes élastiques entrecroisées avec une bande inclinée vers le haut et une bande inclinée vers le bas.

La présente invention vise à remédier aux inconvénients mentionnés ci-dessus. Notamment, un but de l'invention est de fournir une ceinture possédant la forme spécifique requise pour le confort de port et l'efficacité du maintien, mais qui soit d'une réalisation simplifiée et moins onéreuse.

A cet effet, l'invention concerne une ceinture de soutien lombaire qui présente un axe de symétrie médian vertical et un axe longitudinal, et qui comporte deux branches destinées à être positionnées de part et d'autre de la partie inférieure du tronc d'un porteur. Chaque branche présente une partie extrême médiale et une partie extrême latérale, les parties extrêmes médiales étant liées l'une à l'autre dans la zone médiane de la ceinture et les parties extrêmes latérales étant pourvues de moyens d'accrochage complémentaires pour pouvoir être assemblées lorsqu'elles sont placées l'une sur l'autre en regard de la zone abdominale du porteur. La ceinture, lorsqu'elle est à plat et à l'état non contraint, est telle que chaque branche comporte :
- une première bande élastique qui s'étend de la partie extrême latérale à la partie extrême médiale de la branche, selon une direction générale inclinée vers le haut d'un angle α par rapport à l'axe longitudinal de la ceinture ;
- une deuxième bande élastique qui s'étend de la partie extrême latérale à la partie extrême médiale de la branche, selon une direction générale inclinée vers le bas d'un angle β par rapport à l'axe longitudinal de la ceinture ;
les première et deuxième bandes étant superposées, sur une partie de leur surface, selon la direction antéro-postérieure.

De plus, les première et deuxième bandes de chaque branche sont configurées et agencées avec le coin latéral inférieur de la deuxième bande décalé verticalement vers le haut par rapport au coin latéral inférieur de la première bande, de sorte que, lorsque la ceinture est à plat et à l'état non contraint, la branche présente un bord inférieur non rectiligne formé successivement, depuis la partie extrême latérale vers la partie extrême médiale par :
- une portion inclinée vers le haut formée par une partie du bord inférieur de la première bande ;
- puis une portion inclinée vers le bas formée par une partie du bord inférieur de la deuxième bande ;
le bord inférieur de la branche présentant ainsi une échancrure inférieure configurée pour être située au-dessus d'une cuisse du porteur.

Ainsi, dans la ceinture selon l'invention, il est simplement nécessaire de superposer des bandes et de les assembler selon une configuration particulière conférant la géométrie spécifique voulue, en particulier la présence des échancrures inférieures. Aucune opération de découpe des bandes ou de bordage n'étant nécessaire à la formation des échancrures, la réalisation de la ceinture est simple et peu coûteuse, sans toutefois que le confort de port ni l'efficacité de la contention ne soient dégradés. Outre le gain financier, le fait de ne pas avoir à découper les bandes pour former les échancrures garantit également l'intégrité des textiles.

En pratique, les angles α et β sont non nuls et de préférence inférieurs à 60°.

Le terme « coin » s'entend comme la partie de la surface de la bande située au voisinage immédiat du bord latéral et du bord inférieur ; ce coin ne forme pas nécessairement une zone d'angle.

Par ailleurs, on note que les parties extrêmes médiales, si elles sont liées l'une à l'autre, ne le sont pas nécessairement directement (mais éventuellement via une pièce intermédiaire) et/ou ne sont pas nécessairement liées de façon solidaire (mais avec une possibilité de réglage relatif par exemple).

On peut également prévoir que les première et deuxième bandes de chaque branche soient configurées et agencées avec le coin latéral supérieur de la deuxième bande décalé verticalement vers le haut par rapport au coin latéral supérieur de la première bande, de sorte que, lorsque la ceinture est à plat et à l'état non contraint, la branche présente un bord supérieur non rectiligne formé successivement, depuis la partie extrême latérale vers la partie extrême médiale par :
- une portion inclinée vers le bas formée par une partie du bord supérieur de la deuxième bande ;
- puis une portion inclinée vers le haut formée par une partie du bord supérieur de la première bande ;
le bord supérieur de la branche présentant ainsi une échancrure supérieure sensiblement au droit de l'échancrure inférieure le long de la direction verticale.

Une telle configuration améliore encore le confort en position assise en limitant l'appui sur les côtes du porteur.

Selon une réalisation possible, chacune des bandes présente un bord supérieur et un bord inférieur qui sont sensiblement rectilignes et parallèles entre eux. En d'autres termes, la bande a une hauteur constante Dans ce cas, le bord inférieur de la branche forme une ligne brisée, les portions définissant l'échancrure étant des segments.

Les première et deuxième bandes d'une branche peuvent être sensiblement identiques.

L'angle α et l'angle β sont supérieurs à 5°, de préférence supérieurs à 10°, et inférieurs à 30°, de préférence inférieurs à 20°. Par exemple, l'angle α et/ou l'angle β peut être voisin de 12 à 15°.

On peut prévoir que les angles α et β soient sensiblement identiques (en valeur absolue). Si, de plus, les bandes sont identiques, l'axe longitudinal peut alors être un axe de symétrie de la ceinture.

Selon une réalisation possible, l'échancrure inférieure est située dans le tiers central de la branche correspondante, considéré parallèlement à l'axe longitudinal de la ceinture.

En outre, on peut prévoir que l'échancrure inférieure forme un V aplati ouvert vers le bas dont l'angle est compris entre 135 et 170°, de préférence entre 140 et 160°, voire même entre 150 et 160°.

Le décalage vertical entre les coins latéraux inférieurs et/ou supérieurs des bandes peut être compris entre 5 et 30 % de la hauteur d'une bande ou de chacune des bandes, de préférence entre 10 et 25 %, encore de préférence entre 15 et 20 %.

Par exemple, les première et deuxième bandes d'une branche peuvent présenter une même hauteur constante.

Selon une réalisation possible, les bords latéraux des première et deuxième bandes d'une branche sont assemblés l'un à l'autre de sorte à être partiellement superposés le long d'une ligne qui est de préférence sensiblement verticale.

En d'autres termes, ces bords latéraux sont confondus, du moins sur une partie de leur longueur. La superposition partielle - en d'autres termes le décalage relatif des bords latéraux - permet de créer l'échancrure inférieure. Les bords latéraux peuvent être liés à une patte d'extrémité qui porte les moyens d'accrochage sur l'autre branche (elle-même éventuellement pourvue d'une patte d'extrémité).

Ladite ligne, au lieu d'être strictement verticale, pourrait être inclinée par rapport à la verticale, par exemple d'un angle inférieur à 20°.

En outre, la ceinture peut comporter un dossard destiné à être placé contre le dos du porteur, les bords médiaux des première et deuxième bandes d'une branche étant assemblés au dossard. Ils peuvent être assemblés au dossard de façon amovible ; ainsi, lesdits bords médiaux sont repositionnables par rapport au dossard, ce qui permet de faire évoluer la hauteur de traitement, ou de mieux cibler la zone de compression.

Selon une réalisation possible, les bords médiaux des premières bandes des branches sont assemblés l'un à l'autre au niveau d'une première zone de jonction, et les bords médiaux des deuxièmes bandes des branches sont assemblés l'un à l'autre au niveau d'une deuxième zone de jonction, lesdites première et deuxième zones de jonction étant distinctes et assemblées au dossard, de préférence de façon amovible.

Les bords médiaux des première et deuxième bandes d'une branche peuvent être assemblés sur le dossard, de préférence le long d'une ligne qui est par exemple sensiblement confondue avec l'axe de symétrie médian vertical de la ceinture.

Selon une configuration symétrique, qui peut être une configuration standard mais peut être modifiable, les bords médiaux desdites première et deuxième bandes de la branche sont non superposés mais sensiblement adjacents. En d'autres termes, le coin médial inférieur de la première bande est situé juste au-dessus du coin médial supérieur de la deuxième bande.

Selon un mode de réalisation, les bords médiaux de la première bande de la première branche et de la première bande de la deuxième branche peuvent être assemblés au dossard sensiblement le long de la même ligne verticale médiane. De façon similaire, les bords médiaux de la deuxième bande de la première branche et de la deuxième bande de la deuxième branche peuvent être assemblés au dossard sensiblement le long de la même ligne verticale médiane.

Les première et deuxième bandes d'une branche peuvent être superposées, selon la direction antéro-postérieure, de sorte que ladite branche forme une paroi continue dépourvue d'espace vertical entre les première et deuxième bandes, au moins sur la partie de la branche disjointe du dossard. En d'autres termes, au niveau du dossard, il peut exister un tel espace vertical.

On décrit à présent, à titre d'exemples non limitatifs, plusieurs modes de réalisation possibles de l'invention, en référence aux figures annexées :
La figure 2 est une vue postérieure d'une ceinture de soutien lombaire selon l'invention, à plat et à l'état non contraint ;
La figure 3 est une vue antérieure de la ceinture de la figure 2, à plat et à l'état non contraint ;
Les figures 4 et 5 montrent une personne, respectivement de face et de profil, portant la ceinture de la figure 2 ;
La figure 6 est une vue similaire à la figure 2, montrant une variante de la ceinture.

La figure 2 illustre une ceinture de soutien lombaire 1 selon l'invention, lorsqu'elle est posée à plat et est à l'état non contraint, c'est-à-dire en particulier lorsqu'aucune traction n'est exercée sur elle.

La ceinture 1 présente un axe de symétrie médian vertical 2 et un axe longitudinal 3, qui dans certaines configurations de la ceinture 1 peut être un axe de symétrie.

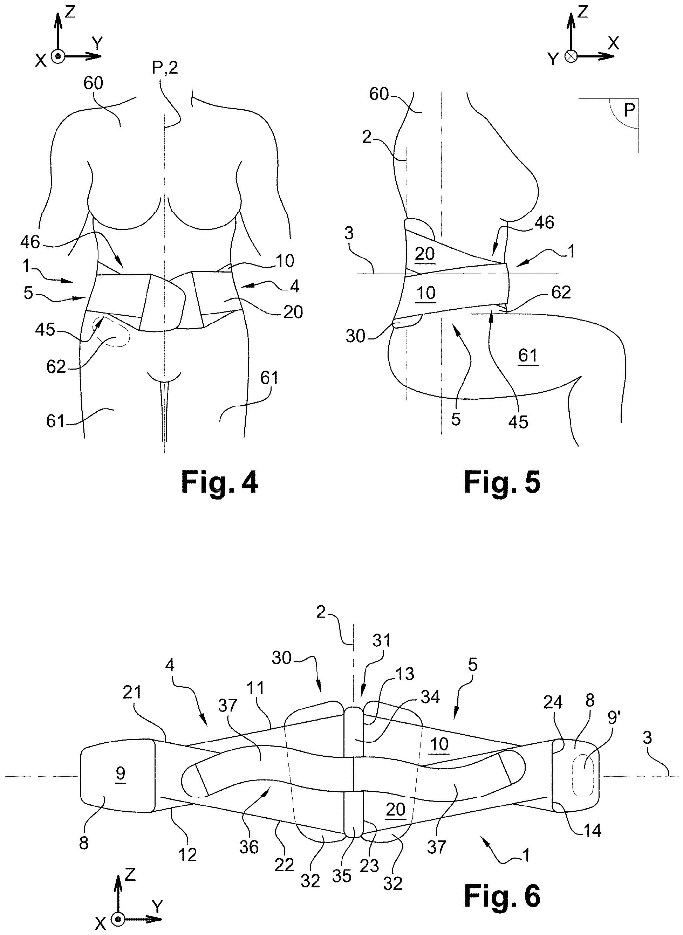

Comme illustré sur les figures 4 et 5, en référence à un porteur 60 de la ceinture 1, et conformément au système de référence anatomique, on définit la direction Z comme la direction verticale, la direction X comme la direction antéro-postérieure, et la direction Y comme la direction horizontale orthogonale à X. Le porteur 60 présente en outre un plan médian P parallèle à (X,Z).

Ainsi, lorsque la ceinture 1 est portée, l'axe de symétrie médian vertical 2 est sensiblement vertical et situé dans le plan P, et l'axe longitudinal 3 - alors enroulé autour du porteur 60 - est sensiblement horizontal.

Les termes « haut », « bas », « hauteur », « inférieur », « supérieur » et analogue sont employés en référence à la direction Z.

Les termes « gauche », « droit », « médial » et « latéral » sont employés en référence à la direction Y. Un élément sera qualifié de « médial » s'il est situé plus près de l'axe de symétrie médian vertical 2 ou du plan P qu'un autre élément, alors qualifié de « latéral ».

Les termes « antérieur », « postérieur » et analogue, sont employés en référence à la direction X.

La ceinture 1 comprend deux branches destinées à être positionnées de part et d'autre de la partie inférieure du tronc d'un porteur, à savoir une branche gauche 4 et une branche droite 5. Chaque branche 4, 5 présente une partie extrême médiale 6 et une partie extrême latérale 7.

Les parties extrêmes médiales 6 des branches 4, 5 sont liées l'une à l'autre dans la zone médiane de la ceinture 1, c'est-à-dire la zone située au centre de la ceinture 1 lorsque celle-ci est posée à plat, cette zone correspondant à la zone dorsale du porteur 60 en position d'utilisation.

Les parties extrêmes latérales 7 des branches 4, 5 sont pourvues de moyens d'accrochage complémentaires 9, 9' pour pouvoir être assemblées lorsqu'elles sont placées l'une sur l'autre en regard de la zone abdominale du porteur 60, comme illustré sur la figure 4.

Selon une réalisation possible, les parties extrêmes latérales 7 des branches 4, 5 comportent chacune une patte d'extrémité 8 qui porte lesdits moyens d'accrochage complémentaires. Par exemple, la face postérieure d'une patte d'extrémité 8 peut comporter une zone 9 de boucles et la face antérieure de l'autre patte d'extrémité 8 peut comporter une zone 9' de crochets, pour former un accrochage de type Velcro ®. Les pattes d'extrémité 8 peuvent être extensibles ou non. Au moins l'une des pattes d'extrémité 8 peut comporter un passe-doigts 33 permettant de faciliter la traction des branches 4, 5 par le porteur 60 lors de la mise en place de la ceinture 1.

La ceinture 1 peut également comprendre un dossard 30 destiné à être placé contre le dos du porteur 60, comme on le voit sur la figure 5.

Le dossard 30 peut être formé d'une partie centrale 31 s'étendant le long de l'axe de symétrie médian vertical 2, destinée à venir au droit de la colonne vertébrale du porteur 60, et de deux parties latérales 32 destinée à être placées de part et d'autre de la colonne vertébrale. Le dossard 30 peut être entièrement rigide ; en variante, les deux parties latérales 32 peuvent être rigides tandis que la partie centrale 31 peut être souple. Les parties latérales 32 peuvent être en saillie vers l'avant par rapport à la partie centrale 31, pour former une gouttière verticale de réception de la colonne vertébrale. En outre, le dossard 30 peut comporter des éléments anti glisse et/ou des éléments de rigidification, et/ou des moyens favorisant la respirabilité (par exemple grâce au matériau constitutif du dossard 30 et/ou à des orifices appropriés).

Chaque branche 4, 5 de la ceinture 1 comporte deux bandes élastiques qui sont superposées, sur une partie de leur surface, selon la direction antéro-postérieure X. Ainsi, lorsque la ceinture 1 est à plat et à l'état non contraint, on a :
- une première bande élastique 10 qui s'étend de la partie extrême latérale 7 à la partie extrême médiale 6 de la branche, selon une direction générale A1 inclinée vers le haut d'un angle α par rapport à l'axe longitudinal 3 de la ceinture 1 ;
- une deuxième bande élastique 20 qui s'étend de la partie extrême latérale 7 à la partie extrême médiale 6 de la branche, selon une direction générale A2 inclinée vers le bas d'un angle β par rapport à l'axe longitudinal de la ceinture 1.

Selon un mode de réalisation, les bandes 10, 20 sont identiques, et sensiblement rectangulaires. Chaque bande possède donc un bord supérieur et un bord inférieur qui sont sensiblement rectilignes et parallèles entre eux, et présente une hauteur h - c'est-à-dire la distance entre le bord inférieur et le bord supérieur, globalement selon la direction Z - sensiblement constante. Plus spécifiquement :
- la première bande 10 comporte un bord supérieur 11, un bord inférieur 12, un bord médial 13 et un bord latéral 14 ;
- la deuxième bande 20 comporte un bord supérieur 21, un bord inférieur 22, un bord médial 23 et un bord latéral 24.

Du fait de l'inclinaison des bandes 10, 20 par rapport à l'axe longitudinal 3 de la ceinture 1, le bord médial et le bord latéral d'une bande, qui peuvent être orientés verticalement, peuvent ne pas être exactement orthogonaux aux bords supérieur et inférieur.

Les bandes 10, 20 peuvent être réalisées en un textile élastique de nervosité appropriée. Elles peuvent être obtenues par découpe à la longueur souhaitée d'une longue bande rectangulaire, transversalement à ladite bande, ce qui ne génère pas de travail de confection important. En revanche, aucune découpe n'est nécessaire au niveau des bords inférieur ni supérieur des bandes 10, 20 pour obtenir la forme recherchée, comme cela est expliqué ci-après.

Les bandes 10, 20 d'une branche 4, 5 donnée sont assemblées l'une à l'autre par leurs bords latéraux 14, 24, qui peuvent par exemple être cousus sur la patte d'extrémité 8 correspondante, de préférence le long d'une ligne sensiblement verticale.

En outre, les bords médiaux 13, 23 des première et deuxième bandes 10, 20 de chaque branche 4, 5 sont assemblés au dossard 30.

Selon une réalisation possible, comme illustré sur la figure 2, les bords médiaux 13 des premières bandes 10 des deux branches 4, 5 sont assemblés l'un à l'autre au niveau d'une première zone de jonction 34, et les bords médiaux 23 des deuxièmes bandes 20 des deux branches 4, 5 sont assemblés l'un à l'autre au niveau d'une deuxième zone de jonction 35. Typiquement, les bords médiaux 13, 23 peuvent être cousus sur la zone de jonction 34, 35 correspondante. Les zones de jonction 34, 35 sont distinctes et assemblées au dossard 30, de préférence de façon amovible. Ces zones de jonction 34, 35 peuvent par exemple comporter, sur leur face antérieure, des moyens d'accrochage amovible sur le dossard 30. L'accrochage peut se faire au niveau de la partie centrale 31 du dossard 30, le long d'une ligne qui est par exemple sensiblement confondue avec l'axe de symétrie médian vertical 2 de la ceinture 1.

Ainsi, de façon concrète, il est possible de détacher une zone de jonction 34, 35 du dossard 30, puis de l'assembler à nouveau au dossard 30 en une position différente. Ceci permet d'augmenter ou de diminuer le ou les angles a, β d'inclinaison des bandes 10, 20, selon les besoins du porteur 60. La symétrie des branches 4, 5 par rapport à l'axe de symétrie médian vertical 2 de la ceinture 1 peut toutefois être conservée.

En variante, les bords médiaux 13, 23 des bandes 10, 20 pourraient être cousus directement sur le dossard 30, sans possibilité de réglage de la hauteur d'assemblage.

Dans la réalisation représentée sur la figure 2, les bandes 10, 20 présentent une même hauteur h constante, qui est d'environ la moitié de la hauteur du dossard 30. Cette réalisation n'est toutefois pas limitative.

En outre, sur la figure 2, les zones de jonction 34, 35 sont assemblées au dossard 30 l'une au-dessus de l'autre, de façon adjacente mais sans superposition. On a alors une ceinture 1 symétrique par rapport à l'axe longitudinal 3, les angles α et β étant identiques, et les pattes d'extrémité 8 étant centrées verticalement par rapport au dossard 30. Cette configuration peut cependant être modifiée par déplacement des zones de jonction 34, 35 par rapport au dossard 30.

Selon une caractéristique de la ceinture 1 selon l'invention, les bandes 10, 20 de chaque branche 4, 5 sont assemblées de sorte que le coin latéral inférieur 25 de la deuxième bande 20 est décalé verticalement vers le haut par rapport au coin latéral inférieur 15 de la première bande 10. De façon concrète, ceci peut être obtenu par couture des bandes 10, 20 à des hauteurs différentes sur la patte d'extrémité 8 correspondante.

Ainsi, lorsque la ceinture 1 est à plat et à l'état non contraint, comme on le voit sur les figures 2 et 3, chaque branche 4, 5 présente un bord inférieur 42, 52 non rectiligne formé successivement, depuis la partie extrême latérale 7 vers la partie extrême médiale 6, par :
- une portion inclinée vers le haut formée par une partie du bord inférieur 12 de la première bande 10 ;
- puis une portion inclinée vers le bas formée par une partie du bord inférieur 22 de la deuxième bande 20.

Il s'ensuit que le bord inférieur 42, 52 de la branche 4, 5 présente une échancrure inférieure 45. En outre, la ceinture 1 est configurée pour que, lorsqu'elle est portée, chacune des échancrures inférieures 45 soit située au-dessus d'une cuisse 61 du porteur 60, c'est-à-dire dans la région inguinale 62 du porteur 60, comme illustré sur les figures 4 et 5.

Grâce à cela, lorsque le porteur 60 s'assoit, la partie supérieure de ses cuisses 61 peut venir se loger dans une échancrure inférieure 45. Ainsi, d'une part, la gêne est considérablement limitée pour le porteur dans la position assise. D'autre part, le risque que la ceinture 1 remonte est significativement diminué, car les cuisses 61 ne viennent pas en appui contre le bord inférieur de la ceinture 1 et ne tendent donc pas à la pousser vers le haut.

Sur la figure 2, la position des cuisses 61 du porteur 60 en position assise est schématisée par une ligne en pointillée.

En pratique, l'échancrure inférieure 45 est de préférence située dans le tiers central de la branche 4, 5 correspondante, considéré parallèlement à l'axe longitudinal 3 de la ceinture 1. De la sorte, une fois la ceinture 1 mise en place sur le porteur 60, l'échancrure 45 inférieure se retrouvera dans la position appropriée au-dessus d'une cuisse 61.

Le décalage vertical d entre le coin latéral inférieur 25 de la deuxième bande 20 et le coin latéral inférieur 15 de la première bande 10 (voir figure 3) peut être compris entre 5 et 30 % de la hauteur h des bandes 10, 20, de préférence entre 10 et 25 %, encore de préférence entre 15 et 20 %.

Par ailleurs, l'angle α et l'angle β peuvent être supérieurs à 5°, de préférence supérieurs à 10°, et inférieurs à 30°, de préférence inférieurs à 20°.

Dans la réalisation représentée, l'échancrure 45 forme une ligne brisée, dans la mesure où les bords inférieurs 12, 22 des première et deuxième bandes 10, 20 sont rectilignes. Ainsi, l'échancrure 45 forme un V aplati ouvert vers le bas. A titre d'exemple, l'angle formé par ce V peut être compris entre 135 et 170°, de préférence entre 140 et 160°, voire même entre 150 et 160°.

On peut par ailleurs prévoir que le coin latéral supérieur 26 de la deuxième bande 20 soit décalé verticalement vers le haut par rapport au coin latéral supérieur 16 de la première bande 10. Ceci est automatiquement le cas si les première et deuxième bandes 10, 20 ont une même hauteur et que leurs coins latéraux inférieurs 15, 25 sont décalés comme décrit précédemment. Le décalage d est alors le même entre les coins latéraux inférieurs 15, 25 et entre les coins latéraux supérieurs 16, 26.

Ainsi, lorsque la ceinture 1 est à plat et à l'état non contraint, chaque branche 4, 5 présente un bord supérieur 41, 51 non rectiligne formé successivement, depuis la partie extrême latérale 7 vers la partie extrême médiale 6, par :
- une portion inclinée vers le bas formée par une partie du bord supérieur 21 de la deuxième bande 20 ;
- puis une portion inclinée vers le haut formée par une partie du bord supérieur 11 de la première bande 10.

En conséquence, le bord supérieur 41, 51 de la branche 4, 5 présente une échancrure supérieure 46. Cette dernière est située sensiblement au droit de l'échancrure inférieure 45 le long de la direction verticale Z, la position relative des échancrures 45, 46 pouvant légèrement varier selon les angles α et β, notamment du fait du réglage en hauteur de la position des zones de jonction 34, 35 sur le dossard 30.

Comme on le voit sur les figures 4 et 5, l'échancrure supérieure 46 permet d'améliorer encore le confort de port, tout particulièrement en position assise, en limitant l'appui sur les côtes du porteur 60.

Quelle que soit la position d'assemblage des zones de jonction 34, 35 sur le dossard 30, il est préférable de prévoir que les première et deuxième bandes 10, 20 de chacune des branches 4, 5 soient superposées, selon la direction antéro-postérieure X, afin que chacune des branches 4, 5 forme une paroi continue dépourvue d'espace vertical entre les première et deuxième bandes 10, 20, au moins sur la partie de la branche 4, 5 disjointe du dossard 30. Ceci répond à des exigences en termes de confort pour le porteur 60 et d'efficacité de la contention.

La figure 6 représente une variante de réalisation de la ceinture 1, qui comporte en plus un dispositif de serrage additionnel 36, par exemple sous la forme de deux sangles latérales 37. Ainsi, les branches 4, 5 peuvent être serrées sans excès et, lorsque cela est nécessaire, il est possible d'augmenter temporairement l'intensité du serrage, typiquement lorsque le porteur 60 va faire un effort important, porter une charge lourde, etc., en tirant les deux sangles latérales 37 et en venant les fixer chacune sur la branche 4, 5 correspondante par des moyens d'accrochage appropriés. Par la suite, il est possible de retourner au serrage initial, de moindre intensité, en détachant le dispositif de serrage additionnel 36.

Ainsi, l'invention apporte une amélioration déterminante à la technique antérieure, en fournissant une ceinture de soutien lombaire de réalisation simple et peu coûteuse permettant néanmoins d'obtenir le confort et l'efficacité requises. Plus spécifiquement, l'échancrure inférieure traditionnellement présente est obtenu simplement par un agencement relatif spécifique des bandes superposées formant chacune des branches de la ceinture. Aucune découpe des bandes au niveau de ses bords inférieur ou supérieur n'est nécessaire, ce qui garantit leur intégrité et simplifie considérablement la confection.

Il va de soi que l'invention n'est pas limitée aux modes de réalisation décrits ci-dessus à titre d'exemples mais qu'elle est définie par les revendications.

## Revendications

1. Ceinture (1) de soutien lombaire présentant un axe de symétrie médian vertical (2) et un axe longitudinal (3), la ceinture (1) comportant deux branches (4, 5) destinées à être positionnées de part et d'autre de la partie inférieure du tronc d'un porteur (60), les branches (4, 5) présentant chacune une partie extrême médiale (6) et une partie extrême latérale (7), les parties extrêmes médiales (6) étant liées l'une à l'autre dans la zone médiane de la ceinture (1) et les parties extrêmes latérales (7) étant pourvues de moyens d'accrochage complémentaires (9, 9') pour pouvoir être assemblées lorsqu'elles sont placées l'une sur l'autre en regard de la zone abdominale du porteur (60), la ceinture (1), lorsqu'elle est à plat et à l'état non contraint, étant telle que chaque branche (4, 5) comporte :
- une première bande (10) élastique qui s'étend de la partie extrême latérale (7) à la partie extrême médiale (6) de la branche (4, 5), selon une direction générale (A1) inclinée vers le haut d'un angle α par rapport à l'axe longitudinal (3) de la ceinture (1) ;
- une deuxième bande (20) élastique qui s'étend de la partie extrême latérale (7) à la partie extrême médiale (6) de la branche (4, 5), selon une direction générale (A2) inclinée vers le bas d'un angle β par rapport à l'axe longitudinal (3) de la ceinture (1) ;
les première et deuxième bandes (10, 20) étant superposées, sur une partie de leur surface, selon la direction antéro-postérieure (X) ;
**caractérisée en ce que** les première et deuxième bandes (10, 20) de chaque branche (4, 5) sont configurées et agencées avec le coin latéral inférieur (25) de la deuxième bande (20) décalé verticalement vers le haut par rapport au coin latéral inférieur (15) de la première bande (10), de sorte que, lorsque la ceinture (1) est à plat et à l'état non contraint, la branche (4, 5) présente un bord inférieur (42, 52) non rectiligne formé successivement, depuis la partie extrême latérale (7) vers la partie extrême médiale (6) par :
- une portion inclinée vers le haut formée par une partie du bord inférieur (12) de la première bande (10) ;
- puis une portion inclinée vers le bas formée par une partie du bord inférieur (22) de la deuxième bande (20) ;
le bord inférieur (42, 52) de la branche (4, 5) présentant ainsi une échancrure inférieure (45) configurée pour être située au-dessus d'une cuisse (61) du porteur (60).

2. Ceinture selon la revendication 1, **caractérisée en ce que** les première et deuxième bandes (10, 20) de chaque branche (4, 5) sont configurées et agencées avec le coin latéral supérieur (26) de la deuxième bande (20) décalé verticalement vers le haut par rapport au coin latéral supérieur (16) de la première bande (10), de sorte que, lorsque la ceinture (1) est à plat et à l'état non contraint, la branche (4, 5) présente un bord supérieur (41, 51) non rectiligne formé successivement, depuis la partie extrême latérale (7) vers la partie extrême médiale (6) par :
- une portion inclinée vers le bas formée par une partie du bord supérieur (21) de la deuxième bande (20) ;
- puis une portion inclinée vers le haut formée par une partie du bord supérieur (11) de la première bande (10) ;
le bord supérieur (41, 51) de la branche (4, 5) présentant ainsi une échancrure supérieure (46) sensiblement au droit de l'échancrure inférieure (45) le long de la direction verticale (Z).

3. Ceinture selon la revendication 1 ou 2, **caractérisée en ce que** chacune des bandes (10, 20) présente un bord supérieur (11, 21) et un bord inférieur (12, 22) qui sont sensiblement rectilignes et parallèles entre eux.

4. Ceinture selon l'une des revendications 1 à 3, **caractérisée en ce que** les première et deuxième bandes (10, 20) d'une branche (4, 5) sont sensiblement identiques.

5. Ceinture selon l'une des revendications 1 à 4, **caractérisée en ce que** l'angle α et l'angle β sont supérieurs à 5°, de préférence supérieurs à 10°, et inférieurs à 30°, de préférence inférieurs à 20°.

6. Ceinture selon l'une des revendications 1 à 5, **caractérisée en ce que** les angles α et β sont sensiblement identiques.

7. Ceinture selon l'une des revendications 1 à 6, **caractérisée en ce que** l'échancrure inférieure (45) est située dans le tiers central de la branche (4, 5) correspondante, considéré parallèlement à l'axe longitudinal (3) de la ceinture (1).

8. Ceinture selon l'une des revendications 1 à 7, **caractérisée en ce que** l'échancrure inférieure (45) forme un V aplati ouvert vers le bas dont l'angle est compris entre 135 et 170°, de préférence entre 140 et 160°, voire même entre 150 et 160°.

9. Ceinture selon l'une des revendications 1 à 8, **caractérisée en ce que** le décalage vertical (d) entre les coins latéraux inférieurs (15, 25) et/ou supérieurs (16, 26) des bandes (10, 20) est compris entre 5 et 30 % de la hauteur (h) d'une bande ou de chacune des bandes (10, 20), de préférence entre 10 et 25 %, encore de préférence entre 15 et 20 %.

10. Ceinture selon l'une des revendications 1 à 9, **caractérisée en ce que** les bords latéraux (14, 24) des première et deuxième bandes (10, 20) d'une branche (4, 5) sont assemblés l'un à l'autre de sorte à être partiellement superposés le long d'une ligne qui est de préférence sensiblement verticale.

11. Ceinture selon l'une des revendications 1 à 10, **caractérisée en ce qu'**elle comporte un dossard (30) destiné à être placé contre le dos du porteur (60), et **en ce que** les bords médiaux (13, 23) des première et deuxième bandes (10, 20) d'une branche (4, 5) sont assemblés au dossard (30).

12. Ceinture selon la revendication 11, **caractérisée en ce que** les bords médiaux (13, 23) des première et deuxième bandes (10, 20) d'une branche (4, 5) sont assemblés sur le dossard (30) de façon amovible.

13. Ceinture selon la revendication 11 ou 12, **caractérisée en ce que** les bords médiaux (13) des premières bandes (10) des branches (4, 5) sont assemblés l'un à l'autre au niveau d'une première zone de jonction (34), et **en ce que** les bords médiaux (23) des deuxièmes bandes (20) des branches (4, 5) sont assemblés l'un à l'autre au niveau d'une deuxième zone de jonction (35), lesdites première et deuxième zones de jonction (34, 35) étant distinctes et assemblées au dossard (30), de préférence de façon amovible.

14. Ceinture selon l'une des revendications 11 à 13, **caractérisée en ce que** les bords médiaux (13, 23) des première et deuxième bandes (10, 20) d'une branche (4, 5) sont assemblés sur le dossard (30), de préférence le long d'une ligne qui est par exemple sensiblement confondue avec l'axe de symétrie médian vertical (2) de la ceinture (1).

15. Ceinture selon l'une des revendications 11 à 14, **caractérisée en ce que** les première et deuxième bandes (10, 20) d'une branche (4, 5) sont superposées, selon la direction antéro-postérieure (X), de sorte que ladite branche (4, 5) forme une paroi continue dépourvue d'espace vertical entre les première et deuxième bandes (10, 20), au moins sur la partie de la branche (4, 5) disjointe du dossard (30).

## Patentansprüche

1. Lendenstützgürtel (1), der eine vertikale Mittensymmetrieachse (2) und eine Längsachse (3) aufweist, wobei der Gürtel (1) zwei Fortsätze (4, 5) beinhaltet, die dazu bestimmt sind, beiderseits des unteren Teils des Rumpfes eines Trägers (60) positioniert zu werden, wobei die Fortsätze (4, 5) jeweils ein mittleres Endteil (6) und ein seitliches Endteil (7) aufweisen, wobei die mittleren Endteile (6) im Mittenbereich des Gürtels (1) miteinander verbunden sind, und die seitlichen Endteile (7) mit ergänzenden Einrastmitteln (9, 9') versehen sind, um zusammengesetzt werden zu können, wenn sie gegenüber dem Abdomen-Bereich des Trägers (60) aufeinander platziert werden, wobei der Gürtel (1), wenn er flach und im zwanglosen Zustand ist, dergestalt ist, dass jeder Fortsatz (4, 5) Folgendes beinhaltet:
- ein erstes elastisches Band (10), das sich von dem seitlichen Endteil (7) zum mittleren Endteil (6) des Fortsatzes (4, 5) in einer um einen Winkel α nach oben geneigten allgemeinen Richtung (A1) in Bezug auf die Längsachse (3) des Gürtels (1) erstreckt;
- ein zweites elastisches Band (20), das sich von dem seitlichen Endteil (7) zum mittleren Endteil (6) des Fortsatzes (4, 5) in einer um einen Winkel β nach unten geneigten allgemeinen Richtung (A2) in Bezug auf die Längsachse (3) des Gürtels (1) erstreckt;
wobei das erste und zweite Band (10, 20) auf einem Teil ihrer Oberfläche in einer Richtung von vorne nach hinten (X) übereinandergelegt sind;
**dadurch gekennzeichnet, dass** das erste und zweite Band (10, 20) jedes Fortsatzes (4, 5) konfiguriert, und mit der seitlichen unteren Ecke (25) des zweiten Bandes (20) in Bezug auf die seitliche untere Ecke (15) des ersten Bandes (10) vertikal nach oben versetzt angeordnet sind, sodass, wenn der Gürtel (1) flach und im zwanglosen Zustand ist, der Fortsatz (4, 5) einen nicht geradlinigen unteren Rand (42, 52) aufweist, der von dem seitlichen Endteil (7) zum mittleren Endteil (6) aufeinanderfolgend gebildet wird durch:
- einen nach oben geneigten Abschnitt, der durch einen Teil des unteren Randes (12) des ersten Bandes (10) gebildet wird;
- danach einen nach unten geneigten Abschnitt, der durch einen Teil des unteren Randes (22) des zweiten Bandes (20) gebildet wird;
wobei der untere Rand (42, 52) des Fortsatzes (4, 5) somit einen unteren Ausschnitt (45) aufweist, der konfiguriert ist, um sich oberhalb eines Oberschenkels (61) des Trägers (60) zu befinden.

2. Gürtel nach Anspruch 1, **dadurch gekennzeichnet, dass** das erste und zweite Band (10, 20) jedes Fortsatzes (4, 5) konfiguriert, und mit der seitlichen oberen Ecke (26) des zweiten Bandes (20) in Bezug auf die seitliche obere Ecke (16) des ersten Bandes (10) vertikal nach oben versetzt angeordnet sind, sodass, wenn der Gürtel (1) flach und im zwanglosen Zustand ist, der Fortsatz (4, 5) einen nicht geradlinigen oberen Rand (41, 51) aufweist, der von dem seitlichen Endteil (7) zum mittleren Endteil (6) aufeinanderfolgend gebildet wird durch:
- einen nach unten geneigten Abschnitt, der durch einen Teil des oberen Randes (21) des zweiten Bandes (20) gebildet wird;
- danach einen nach oben geneigten Abschnitt, der durch einen Teil des oberen Randes (11) des ersten Bandes (10) gebildet wird;
wobei der obere Rand (41, 51) des Fortsatzes (4, 5) somit einen oberen Ausschnitt (46) im Wesentlichen auf Höhe des unteren Ausschnitts (45) entlang der vertikalen Richtung (Z) aufweist.

3. Gürtel nach Anspruch 1 oder 2, **dadurch gekennzeichnet, dass** jedes der Bänder (10, 20) einen oberen Rand (11, 21) und einen unteren Rand (12, 22) aufweist, die im Wesentlichen geradlinig und parallel zueinander sind.

4. Gürtel nach einem der Ansprüche 1 bis 3, **dadurch gekennzeichnet, dass** das erste und zweiten Band (10, 20) eines Fortsatzes (4, 5) im Wesentlichen identisch sind.

5. Gürtel nach einem der Ansprüche 1 bis 4, **dadurch gekennzeichnet, dass** der Winkel α und der Winkel β größer als 5°, vorzugsweise größer als 10° und kleiner als 30°, vorzugsweise kleiner als 20° sind.

6. Gürtel nach einem der Ansprühe 1 bis 5, **dadurch gekennzeichnet, dass** die Winkel α und β im Wesentlichen identisch sind.

7. Gürtel nach einem der Ansprüche 1 bis 6, **dadurch gekennzeichnet, dass** sich der untere Ausschnitt (45) im zentralen Drittel des entsprechenden Fortsatzes (4, 5) befindet, das parallel zur Längsachse (3) des Gürtels (1) angenommen wird.

8. Gürtel nach einem der Ansprüche 1 bis 7, **dadurch gekennzeichnet, dass** der untere Ausschnitt (45) ein abgeflachtes, nach unten offenes V bildet, dessen Winkel zwischen 135 und 170°, vorzugsweise zwischen 140 und 160°, oder gar zwischen 150 und 160° enthalten ist.

9. Gürtel nach einem der Ansprüche 1 bis 8, **dadurch gekennzeichnet, dass** der vertikale Versatz (d) zwischen den unteren (15, 25) und/oder oberen (16, 26) seitlichen Ecken der Bänder (10, 20) zwischen 5 und 30 % der Höhe (h) eines Bandes oder jedes der Bänder (10, 20), vorzugsweise zwischen 10 und 25 %, besonders bevorzugt zwischen 15 und 20 % enthalten ist.

10. Gürtel nach einem der Ansprüche 1 bis 9, **dadurch gekennzeichnet, dass** die seitlichen Ränder (14, 24) des ersten und zweiten Bandes (10, 20) eines Fortsatzes (4, 5) derart miteinander zusammengesetzt sind, um entlang einer Linie, die vorzugsweise im Wesentlichen parallel ist, teilweise überlagert zu sein.

11. Gürtel nach einem der Ansprüche 1 bis 10, **dadurch gekennzeichnet, dass** er eine Rückenplatte (30) beinhaltet, die dazu bestimmt ist, am Rücken des Trägers (60) platziert zu werden, und dadurch, dass die mittleren Ränder (13, 23) des ersten und zweiten Bandes (10, 20) eines Fortsatzes (4, 5) mit der Rückenplatte (30) zusammengesetzt sind.

12. Gürtel nach Anspruch 11, **dadurch gekennzeichnet, dass** die mittleren Ränder (13, 23) des ersten und zweiten Bandes (10, 20) eines Fortsatzes (4, 5) auf abnehmbare Weise auf der Rückenplatte (30) zusammengesetzt sind.

13. Gürtel nach Anspruch 11 oder 12, **dadurch gekennzeichnet, dass** die mittleren Ränder (13) der ersten Bänder (10) der Fortsätze (4, 5) im Bereich einer ersten Verbindungszone (34) miteinander zusammengesetzt sind, und dadurch, dass die mittleren Ränder (23) der zweiten Bänder (20) der Fortsätze (4, 5) im Bereich einer zweiten Verbindungszone (35) miteinander zusammengesetzt sind, wobei die erste und zweite Verbindungszone (34, 35) unterschiedlich sind und vorzugsweise auf abnehmbare Weise mit der Rückenplatte (30) zusammengesetzt sind.

14. Gürtel nach einem der Ansprüche 11 bis 13, **dadurch gekennzeichnet, dass** die mittleren Ränder (13, 23) des ersten und zweiten Bandes (10, 20) eines Fortsatzes (4, 5) vorzugsweise entlang einer Linie, die beispielsweise im Wesentlichen mit der vertikalen Mittensymmetrieachse (2) des Gürtels (1) zusammenfällt, auf der Rückenplatte (30) zusammengesetzt sind.

15. Gürtel nach einem der Ansprühe 11 bis 14, **dadurch gekennzeichnet, dass** das erste und zweite Band (10, 20) eines Fortsatzes (4, 5) in der Richtung von vorne nach hinten (X) überlagert sind, sodass der Fortsatz (4, 5) eine durchgehende Wand bildet, die mindestens an dem Teil des Fortsatzes (4, 5), der von der Rückenplatte (30) getrennt ist, mit keinem vertikalen Zwischenraum zwischen dem ersten und zweiten Band (10, 20) versehen ist.

## Claims

1. A lumbar support belt (1) having a vertical median axis of symmetry (2) and a longitudinal axis (3), the belt (1) including two branches (4, 5) intended to be positioned on either side of the lower portion of the trunk of a wearer (60), the branches (4, 5) each having a medial end portion (6) and a lateral end portion (7), the medial end portions (6) being linked to each other in the median area of the belt (1) and the lateral end portions (7) being provided with complementary attaching means (9, 9') to enable them to be assembled when they are placed on each other in the abdominal area of the wearer (60), the belt (1), when it is flat and in the unstressed state, being such that each branch (4, 5) includes:
- a first elastic strip (10) which extends from the lateral end portion (7) to the medial end portion (6) of the branch (4, 5), according to a general direction (A1) upwardly inclined at an angle α with respect to the longitudinal axis (3) of the belt (1);
- a second elastic strip (20) which extends from the lateral end portion (7) to the medial end portion (6) of the branch (4, 5), according to a general direction (A2) downwardly inclined at an angle β with respect to the longitudinal axis (3) of the belt (1);
the first and second strips (10, 20) being superimposed, over a portion of their surface, in the anterior-posterior direction (X);
**characterized in that** the first and second strips (10, 20) of each branch (4, 5) are configured and arranged with the lower lateral corner (25) of the second strip (20) which is offset vertically upwards with respect to the lower lateral corner (15) of the first strip (10), so that, when the belt (1) is flat and in the unstressed state, the branch (4, 5) has a non-rectilinear lower edge (42, 52) successively formed, from the lateral end portion (7) to the medial end portion (6) by:
- an upwardly inclined portion formed by a portion of the lower edge (12) of the first strip (10);
- then a downwardly inclined portion formed by a portion of the lower edge (22) of the second strip (20);
the lower edge (42, 52) of the strip (4, 5) thus having a lower indentation (45) configured to be located above a thigh (61) of the wearer (60).

2. The belt according to claim 1, **characterized in that** the first and second strips (10, 20) of each branch (4, 5) are configured and arranged with the upper lateral corner (26) of the second strip (20) which is offset vertically upwards with respect to the upper lateral corner (16) of the first strip (10), so that when the belt (1) is flat and in the unstressed state, the branch (4, 5) has a non-rectilinear upper edge (41, 51) successively formed, from the lateral end portion (7) to the medial end portion (6) by:
- a downwardly inclined portion formed by a portion of the upper edge (21) of the second strip (20);
- then an upwardly inclined portion formed by a portion of the upper edge (11) of the first strip (10);
the upper edge (41, 51) of the branch (4, 5) thus having an upper indentation (46) substantially facing the lower indentation (45) along the vertical direction (Z).

3. The belt according to claim 1 or 2, **characterized in that** each of the strips (10, 20) has an upper edge (11, 21) and a lower edge (12, 22) which are substantially rectilinear and parallel to each other.

4. The belt according to any of claims 1 to 3, **characterized in that** the first and second strips (10, 20) of a branch (4, 5) are substantially identical.

5. The belt according to any of claims 1 to 4, **characterized in that** the angle α and the angle β are larger than 5°, preferably larger than 10°, and smaller than 30°, preferably smaller than 20°.

6. The belt according to any of claims 1 to 5, **characterized in that** the angles α and β are substantially identical.

7. The belt according to any of claims 1 to 6, **characterized in that** the lower indentation (45) is located in the central third of the corresponding branch (4, 5), considered parallel to the longitudinal axis (3) of the belt (1).

8. The belt according to any of claims 1 to 7, **characterized in that** the lower indentation (45) forms a downwardly open flattened V whose angle is comprised between 135 and 170°, preferably between 140 and 160°, or even between 150 and 160°.

9. The belt according to any of claims 1 to 8, **characterized in that** the vertical offset (d) between the lower (15, 25) and/or upper (16, 26) lateral corners of the strips (10, 20) is comprised between 5 and 30% of the height (h) of a strip or each of the strips (10, 20), preferably between 10 and 25%, even more preferably between 15 and 20%.

10. The belt according to any of claims 1 to 9, **characterized in that** the lateral edges (14, 24) of the first and second strips (10, 20) of a branch (4, 5) are assembled to each other so as to be partially superimposed along a line which is preferably substantially vertical.

11. The belt according to any of claims 1 to 10, **characterized in that** it includes a back piece (30) intended to be placed against the back of the wearer (60), and **in that** the medial edges (13, 23) of the first and second strips (10, 20) of a branch (4, 5) are assembled to the back piece (30).

12. The belt according to claim 11, **characterized in that** the medial edges (13, 23) of the first and second strips (10, 20) of a branch (4, 5) are removably assembled on the back piece (30).

13. The belt according to claim 11 or 12, **characterized in that** the medial edges (13) of the first strips (10) of the branches (4, 5) are assembled to each other in a first junction area (34), and **in that** the medial edges (23) of the second strips (20) of the branches (4, 5) are assembled to each other in a second junction area (35), said first and second junction areas (34, 35) being distinct and assembled to the back piece (30), preferably removably assembled to the back piece (30).

14. The belt according to any of claims 11 to 13, **characterized in that** the medial edges (13, 23) of the first and second strips (10, 20) of a branch (4, 5) are assembled on the back piece (30), preferably along a line which is for example substantially coincident with the vertical median axis of symmetry (2) of the belt (1).

15. The belt according to any of claims 11 to 14, **characterized in that** the first and second strips (10, 20) of a branch (4, 5) are superimposed, according to the anterior-posterior direction (X), so that said branch (4, 5) forms a continuous wall devoid of a vertical space between the first and second strips (10, 20), at least over the portion of the branch (4, 5) separated from the back piece (30).
